# EUROPEAN PATENT APPLICATION

(11) **EP 1 618 834 A1**
(43) Date of publication of application: **25.01.2006**
(21) Application number: 04728637.2
(22) Date of filing: 21.04.2004
(51) Int. Cl.: A61B 1/06

(54) **CAPSULE ENDOSCOPE AND CAPSULE ENDOSCOPE SYSTEM**

(30) Priority: 25.04.2003 JP 2003122806
(71) Applicant: Olympus Corporation, Tokyo 151-0072 (JP)
(72) Inventor: KIMOTO, Seiichiro, Hachioji-shi, Tokyo 1920045 (JP); FUJIMORI, Noriyuki, Suwa-shi, Nagano 3920131 (JP); SUZUSHIMA, Hiroshi, Kamiina-gun, Nagano 3994601 (JP); SHIGEMORI, Toshiaki, Kawasaki-shi, Kanagawa 2140034 (JP); NAKAMURA, Tsutomu, Kamiina-gun, Nagano 3994511 (JP); NAGASE, Ayako, Hachioji-shi, Tokyo 1920045 (JP); MINAI, Tetsuo, Hachioji-shi, Tokyo 1920023 (JP); SHIMIZU, Hatsuo, Hachioji-shi, Tokyo 1920024 (JP); HONDA, Takemitsu, Hino-shi, Tokyo 1910062 (JP); SASAGAWA, Katsuyoshi, Hino-shi, Tokyo 1910041 (JP); SUZUKI, Katsuya, Sagamihara-shi, Kanagawa 2291103 (JP); HASHIMOTO, Masayuki, Nara-shi, Nara 6310806 (JP); ORIHARA, Tatsuya, Hachioji-shi, Tokyo 1920023 (JP); NAKATSUCHI, Kazutaka, Hino-shi, Tokyo 1910062 (JP)
(74) Representative: von Hellfeld, Axel
(86) International application number: PCT/JP2004/005679
(87) International publication number: WO 2004/096028

(57) **Abstract**

A capsule endoscope includes an image capturing unit (11) having an image capturing section that can capture images of an inside of an abdominal cavity, an illuminating unit (12) that illuminates the inside of the abdominal cavity, a power-supply unit (13) that supplies electric power to both the image capturing unit (11) and the illuminating unit 12, and a capsule casing (14) in which at least the image capturing unit (11), the illuminating unit (12), and the power-supply unit (13) are disposed. A front cover (20) of the capsule casing has an illuminating window (20a) for allowing a light L output by the illuminating unit (12) to pass through and that makes it difficult for the light L to reflect toward the image capturing unit (11).

## Description

### TECHNICAL FIELD

The present invention relates to endoscopes for medical use, and more particularly, relates to a swallowable capsule-shaped endoscope (hereinafter, "capsule endoscope") and a capsule endoscope system.

### BACKGROUND ART

A capsule endoscope which contains an illuminating unit in a capsule casing having a size that a patient can swallow, where the illuminating unit uses, for example, an image capturing unit includes a solid-state image sensor, or an LED, has been known.

Such a capsule endoscope can capture images of the inside of an abdominal cavity such as a stomach and intestines when passing through the abdominal cavity after a patient swallows it.

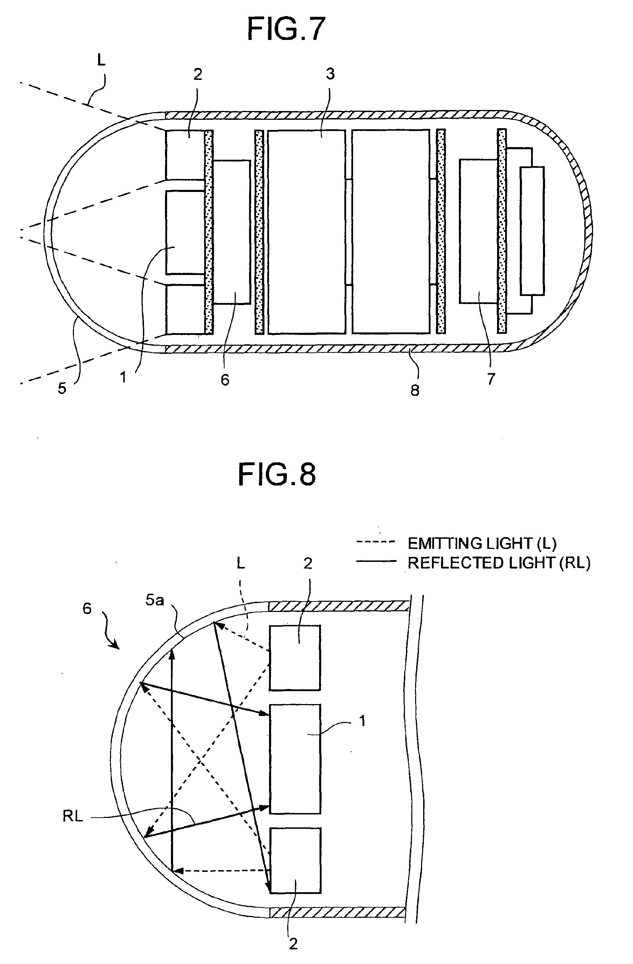

An example of the capsule endoscope is described below with reference to Fig. 7.

As shown in Fig. 7, the conventional capsule endoscope includes an image capturing unit 1 that captures images of the inside of an abdominal cavity, an illuminating unit 2 that illuminates the inside of the abdominal cavity, a power-supply unit 3 that supplies power to these units, a front cover 5 that covers the image capturing unit 1 and the illuminating unit 2 and guides the light L from the illuminating unit 2 to outside, and a capsule casing 8 that is assembled to retain watertight sealing between the capsule casing 8 and front cover 5 and that houses at least the image capturing unit 1, the illuminating unit 2, the power-supply unit 3, and processing circuits 6 and 7.

The illuminating unit 2 can illuminate the inside of the abdominal cavity while the image capturing unit 1 captures images of the inside of the abdominal cavity. The illuminating unit 2 and the image capturing unit 1 are generally disposed near each other in facing forward. Provided at the top of the capsule casing 8 is the front cover 5 having a semispherical shape, serving as an illumination guiding window and an abdominal cavity capturing window, and being partly or fully transparent, to guide the illumination light to the inside of the abdominal cavity and to allow the image capturing unit to capture images of the inside of the abdominal cavity.

The semispherical shape of the front cover 5 allows a patient to easily swallow the capsule endoscope and makes it difficult for body fluids and the like to attach and remain on the front cover 5 (see Japanese Patent Application Laid-open Publication No. 2001-95756).

However, since the conventional semispherical front cover 5 disclosed in Japanese Patent Application Laid-open Publication No. 2001-95756 has the so-called domed shape, an illumination guiding window 5a integrated with the front cover 5 has a curved surface as shown in Fig. 8. As a result, there is a problem that the illuminating light L from the illuminating unit 2 cannot enter the illumination guiding window in the direction perpendicular to the surface, and when the illuminating light L is reflected on the inside of the semispherical cover 5, the reflected light RL enters the image capturing unit 1, which is disposed at the side of the illuminating unit 2.

### DISCLOSURE OF INVENTION

To solve the above-mentioned problems and achieve the above-mentioned objects, the present invention comprises an image capturing unit that has an image capturing section for capturing images of an inside of an abdominal cavity; an illuminating unit that illuminates the inside of the abdominal cavity with illuminating light; a power-supply unit that supplies power to the image capturing unit and the illuminating unit; a front cover that covers the image capturing unit and the illuminating unit, guides the illuminating light from the illuminating unit to outside, and includes an illuminating light guide window making it difficult for the illuminating light to reflect toward the image capturing unit; and a capsule casing that is provided to retain watertight sealing between the capsule casing and the front cover, and houses the image capturing unit, the illuminating unit, and the power-supply unit.

In the invention, there may be a distance between the illuminating light guide window and the illuminating unit, the distance making it difficult for reflected light of the illuminating light from the illuminating unit on the illuminating guide window to enter the image capturing unit.

In the invention, the distance between the illuminating light guide window and the illuminating unit may be 2 millimeter or less.

In the invention, a adjacent distance between the illuminating unit and the image capturing unit may be 1 millimeter or less.

In the invention, the illuminating light guide window can cover almost whole of a light emitting surface of the illuminating unit from front.

In the invention, the illuminating light guide window may be disposed in a direction orthogonal to a central optical axis of the illuminating light from the illuminating unit.

In the invention, the illuminating light guide window may be made of one of resin and glass.

In the invention, a central portion of the front cover other than the illuminating light guide window may be convex.

In the invention, at least two illuminating units may be arranged around the image capturing unit.

The invention may further comprise a communication unit that transmits captured image information from the image capturing unit to outside.

The invention comprises the capsule endoscope; a package that covers the capsule endoscope before the capsule endoscope is used; a receiving unit that receives the information from the capsule endoscope; and an information processing unit that processes the information received from the receiving unit.

### BRIEF DESCRIPTION OF DRAWINGS

Fig. 1 is a schematic diagram of the internal structure of a capsule endoscope of a first embodiment according to the present invention;
Fig. 2 is a schematic diagram of a capsule endoscope system;
Fig. 3 is a schematic diagram of a capsule endoscope according to another embodiment;
Fig. 4 is a perspective view of a front cover;
Fig. 5 is a schematic diagram for explaining emission of illuminating light from an illuminating unit;
Fig. 6 is a schematic diagram for explaining reflection of the illuminating light from the illuminating unit;
Fig. 7 is a schematic diagram of a conventional capsule endoscope; and
Fig. 8 is a schematic diagram for explaining guide for and reflection of illuminating light from an illuminating unit of the conventional capsule endoscope.

### BEST MODE(S) FOR CARRYING OUT THE INVENTION

The present invention will be explained in detail below through embodiments of the invention but is not limited to those embodiments.

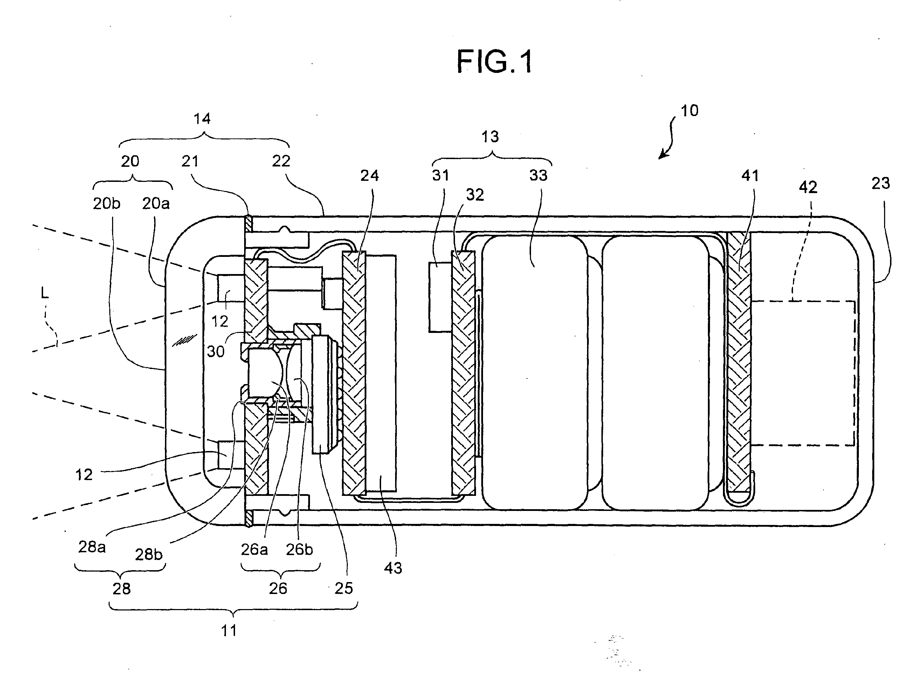

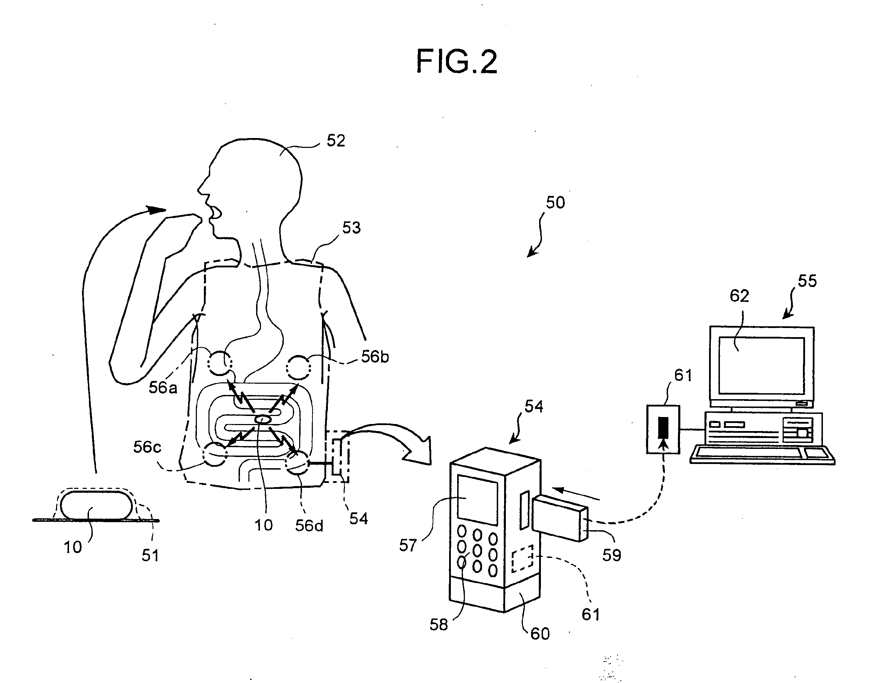

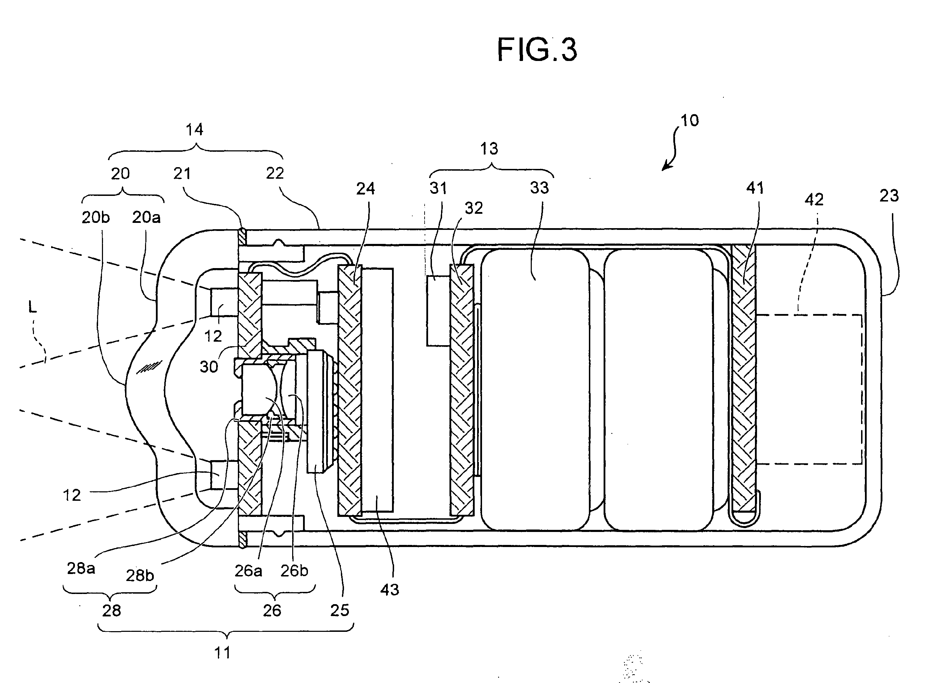

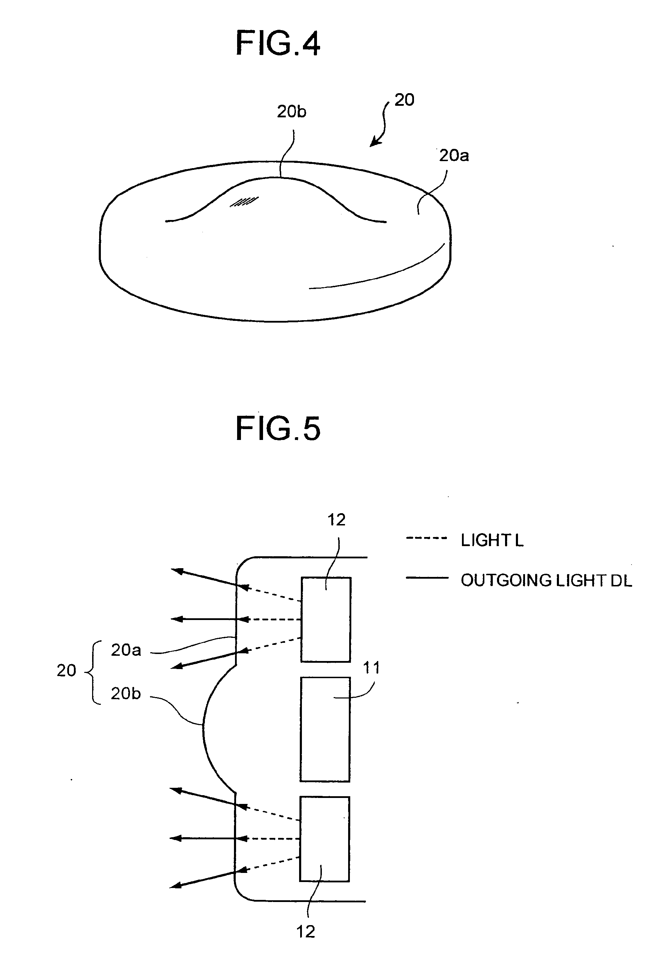

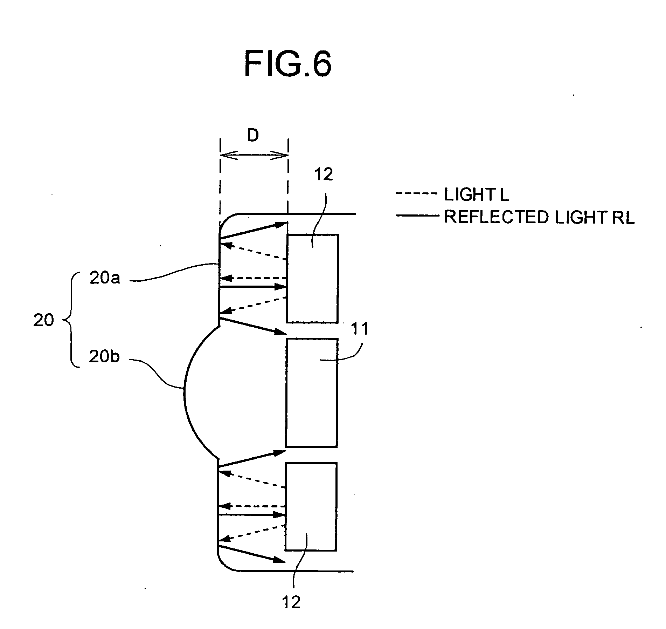

Fig. 1 is a schematic diagram of the internal structure of a capsule endoscope of a first embodiment according to the present invention; Fig. 2 is a schematic diagram of a capsule endoscope system; Fig. 3 is a schematic diagram of a capsule endoscope according to another embodiment; Fig. 4 is a perspective view of a front cover; Fig. 5 is a schematic diagram for explaining emission of illuminating light from an illuminating unit; and Fig. 6 is a schematic diagram for explaining reflection of the illuminating light from the illuminating unit.

The structure of the capsule endoscope according to the present invention is first described with reference to a side view of the internal structure of the capsule endoscope of Fig. 1. As shown in Fig. 1, the capsule endoscope 10 includes an image capturing unit 11 for capturing images of the inside of an abdominal cavity, an illuminating unit 12 that illuminates the inside of the abdominal cavity, a power-supply unit 13 that supplies an electric power to both units, and a watertight capsule casing 14 that houses the image capturing unit 11, the illuminating unit 12, and the power-supply unit 13 at least.

The capsule casing 14 according to this embodiment has a front cover 20 that includes an illuminating window 20a for guiding the illuminating light L from the image capturing unit 11 and the illuminating unit 12 to outside, and preventing reflection of the illuminating light L to the image capturing unit, the front cover 20 that covers the illuminating unit 12, and a capsule trunk 22 that is provided to retain watertight sealing between the capsule trunk 22 and the front cover 20 by a watertight sealer 21 and that houses the image capturing unit 11 and the like. A rear cover, which is separable, if required, may be provided to the capsule trunk 22. It is assumed that the rear cover is provided integrally with the capsule trunk and is flat, however, its shape is not limited to but the rear cover may be dome-shaped, for example.

As shown in Fig. 3, the front cover 20 may be divided to the flat illuminating window 20a for allowing the illumination light L from the illuminating unit 12 to pass through and a convex capturing window 20b for capturing images in an illuminating range. The whole of the front cover 20 is transparent.

The image capturing unit 11 is installed on an image capturing substrate 24, and includes a solid-state image sensor 25 which is, for example, a charged couple device (CCD), and which captures images in the range illuminated by the light L from the illuminating unit 12; and an image forming lens 26, for forming an image of an object on the solid-state image sensor 25, which includes a fixed lens 26a and a movable lens 26b A sharp image is formed by a focusing unit 28 which includes a fixing frame to firmly hold the fixed lens 26a and a movable frame to movably hold the movable lens 26b.

In the present invention, the image capturing unit 11 is not limited to the CCD, but may be another image capturing unit such as a complementary metal-oxide semiconductor (CMOS).

The illuminating unit 12 is provided on an illuminating substrate 30 and is, for example, a light emitting diode (LED). A plurality of illuminating units 12 (four illuminating units in this embodiment) are arranged around the image forming lens 26, which constitutes the image capturing unit 11.

The power-supply unit 13 is installed on a power-supply substrate 32 that has an internal switch 31, and uses, for example, a button battery cell as a power supply 33. It is assumed here that the battery cell is a silver-oxide cell; however, the battery cell may be a rechargeable cell, a dynamo cell, or the like.

The internal switch 31 is a switch that can be made ON by magnets.

In this embodiment, a wireless unit 42, which includes an antenna etc., for performing wireless communication with outside is installed on a wireless substrate 41, and performs communication with the outside if required.

A signal-processing and control unit 43 that processes and controls the various units is installed on the image capturing substrate 24, and executes various processes in the capsule endoscope.

The signal-processing and control unit 43 includes an image-signal processing function such as image-data generation with correlated double sampling (CDS); a transmission-signal generating function to perform, for example, mixing of an image signal and a synchronization signal (in a case of analog transmission) and addition of an error correction code (in a case of digital transmission); a modulation function to convert in cooperation with a modulator, to a phase-shift keying (PSK) modulation, a minimum-shift keying (MSK) modulation, a Gaussian minimum-shift keying (GMSK) modulation, a quadrature minimum-shift keying (QMSK) modulation, and an amplitude-shift keying (ASK) modulation format, for example; a power-supply control function to control the power supply according to ON-OFF operation of a switch; a timing-generator (TG) function to control a driver circuit like an LED driver circuit and to control the number of images to be captured; and a storage function to store various data like parameters of line and frame etc., and performs various signal processing and controls.

The signal processing may include image-data correction (white balance (WB) correction, y correction, color processing, automatic gain control (AGC) etc.), analog-digital conversion (ADC), automatic exposure control function (AE), and the like.

A capsule endoscope system according to the embodiment is explained below with reference to Fig. 2. Fig. 2 is a schematic diagram of the capsule endoscope system according to the present embodiment. The capsule endoscope system shown in Fig. 2 is used for checking a patient with the capsule endoscope 10.

The capsule endoscope system 50 according to this embodiment includes, for example, the capsule endoscope 10, its package 51, a jacket 53 to be worn by a patient 52, a receiver 54 that can be detachably attached to the jacket 53, and a work station 55 that processes information received in the receiver 54.

Antennas 56a, 56b, 56c, and 56d which receive electromagnetic waves of image signals transmitted from the wireless unit 42 of the capsule endoscope 10 are installed in the jacket 52 and are provided to enable wireless communication or wired communication by a cable with the receiver 54. The number of antennas installed in the jacket 53 is not limited to four and would be two or more so that the electromagnetic waves from the location of the capsule endoscope 10, which changes by its movement, can be received properly.

The receiver 54 includes a display 57 that displays information necessary for observation (examination) and an input unit 58 to input information necessary for observation (examination). A CF (compact flash (registered trademark)) memory 59 that stores the received image data, is detachably mounted on the receiver 54. The receiver 54 is also provided with a power-supply unit 60 that can supply power even while carrying, and a signal processing and control section 61 that performs processing required for observation (examination). A dry battery cell, a lithium-ion secondary battery cell, nickel-hydrogen battery cell etc. are examples of the power-supply unit 60 and it may be a rechargeable battery cell as well.

The work station 55 has processing functions to perform diagnosis based on images of internal organs in a body of a patient which a doctor or a nurse has captured by the capsule endoscope 10. This work station 55 is provided with a CF memory reader/writer 61. It is not shown in the diagram but the receiver 54 and the CF memory reader/writer 61 have interfaces that can be connected to enable communication, and read and write the CF memory 59.

Moreover, the work station 55 has a communication function for connecting to a network, and via this network, a medical examination result of the patient is stored in a database. Further, the work station 55 has a display 62 and receives the captured image data of the inside of the patient's body from the receiver 54, and displays images of internal organs etc. on the display 62.

As shown in FIG. 2, before the examination is carried out, the capsule endoscope 10 is taken out from the package 51, and the patient 52 swallows the capsule endoscope 10. The capsule endoscope 10 passes through esophagus of the patient, advances to an abdominal cavity through peristalsis of an alimentary canal cavity, and captures images of the inside of the abdominal cavity one after another.

The result of capturing, when needed or at any time, is output as the electromagnetic waves of the captured images via the wireless unit 42, and is received by the antennas 56a to 56d of the jacket 53. The antenna that receives signals with a high electromagnetic power receiver 54 transmits the signals to the receiver 54 which is located outside the body of the patient.

The receiver 54 stores the captured image data, which is sequentially received, in the CF memory 59. The operation of the receiver 54 is not synchronized with the start of image capturing of the capsule endoscope 10, but, the start and the end of receiving are controlled by an operation of the input unit 58. The captured image data may be still-image data captured at a plurality of frames per second for displaying them as moving images or may be normal video-image data.

When the observation (examination) of the patient 52 by the capsule endoscope 10 is completed, the CF memory 59 is inserted into the CF memory reader/writer 61. The captured data in the CF memory 59 is then transferred to the work station 55. In the work station 55, the transmitted data is associated with and stored for each patient.

Thus, the captured image data of the inside of the abdominal cavity, which is captured by the capsule endoscope 10 and stored by the receiver 54, is displayed by the display 62 of the work station 55. This enables to acquire images of internal organs (e.g., a small intestine) which are not accessible by an ultrasonic probe, endoscope etc.

The front cover is described in detail with reference to Figs. 1, 3, and 6.

As shown in Figs. 1 and 3, the front cover according to this embodiment covers the image capturing unit 11 and the illuminating unit 12. The illuminating window 20a for allowing the illumination light L from the illuminating unit 12 to pass through is formed on the front cover. According to the embodiment shown in Fig. 1, the entire surface of the front cover 20 is flat; according to the embodiment shown in Fig. 3, the capturing window 20b of the front cover 20 has a convex shape.

In both the embodiments, the illuminating window 20a is flat to prevent influence on the image capturing unit 11 due to reflection of the light L from the illuminating unit 12.

Hence, the light L in the optical axis of the illuminating unit 12 enters the illuminating window 20a straight. The reflected light illuminates the abdominal cavity as the outgoing light DL but does not enter the image capturing unit 11, thereby having no influence on the image capturing unit 11 due to the reflection.

Moreover, as the front cover 20 shown in Figs. 3 and 4, a central part is protruded out to form a convex part 21a, and a part other than the convex part is made to be a illuminating light guide window 21b for making it difficult for the flat illuminating light L to reflect toward the image capturing unit, thereby making it easy for the patient 52 to swallow the capsule endoscope 10.

By making the front cover 20 as in the present embodiment, since a central optical axis of the light L from the illuminating unit 12 and the illuminating window 20b are orthogonal to each other as shown in Fig. 5, the reflection in the image forming lens 26 in the image capturing unit 11 is prevented as shown in Fig. 6.

In other words, the light L is incident straight on the window 20a and passes through easily. Thus, since the light L is not reflected to the image capturing unit 11, flare etc. is prevented.

Furthermore, as shown in Fig. 6, regarding a distance D between the illuminating window 20b and the illuminating unit 12, a range of 0.01 mm to 3 mm is desirable, and a range of 0.1 mm to 2 mm is more desirable. If the distance D is more than 3 mm, since, for example, an LED emits radially, it is not favorable.

Moreover, it is desirable that a connecting portion with the capsule trunk 22 of the front cover is R-shaped form. This is because the curve surface eases swallowing by the patient.

Furthermore, when a distance between illuminating unit 12 and the flat window 20a is short, an area of the flat window 20a may be slightly less than an area of a surface of the illuminating unit 12 and when the distance between the two becomes longer, at least the area of the flat window 20a is required to be kept roughly the same as the area of the surface of the illuminating unit 12. This is because as the distance between the illuminating unit 12 and the flat window 20a for illuminating light becomes longer, the reflected light tend to reach towards the image capturing unit 11.

According to the present embodiment, since the window for illuminating the light is made so that illuminating light cannot be reflected easily to an image capturing unit, it is possible to prevent reflection to the image capturing unit thereby enabling to obtain clear images.

Moreover, by making a capsule endoscope system that includes the capsule endoscope, it is possible to make an examination with high accuracy.

### INDUSTRIAL APPLICABILITY

The present invention is applicable to a capsule-shaped endoscope and a capsule endoscope system than can acquire good images of an inside of a body of a patient.

## Claims

1. A capsule endoscope comprising:
an image capturing unit that has an image capturing section for capturing images of an inside of an abdominal cavity;
an illuminating unit that illuminates the inside of the abdominal cavity with illuminating light;
a power-supply unit that supplies power to the image capturing unit and the illuminating unit;
a front cover that covers the image capturing unit and the illuminating unit, guides the illuminating light from the illuminating unit to outside, and includes an illuminating light guide window making it difficult for the illuminating light to reflect toward the image capturing unit; and
a capsule casing that is provided to retain watertight sealing between the capsule casing and the front cover, and houses the image capturing unit, the illuminating unit, and the power-supply unit.

2. The capsule endoscope according to claim 1, wherein there is a distance between the illuminating light guide window and the illuminating unit, the distance making it difficult for reflected light of the illuminating light from the illuminating unit on the illuminating guide window to enter the image capturing unit.

3. The capsule endoscope according to claim 2, wherein the distance between the illuminating light guide window and the illuminating unit is 2 millimeter or less.

4. The capsule endoscope according to claim 2, wherein a adjacent distance between the illuminating unit and the image capturing unit is 1 millimeter or less.

5. The capsule endoscope according to claim 1, wherein the illuminating light guide window can cover almost whole of a light emitting surface of the illuminating unit from front.

6. The capsule endoscope according to claim 1, wherein the illuminating light guide window is disposed in a direction orthogonal to a central optical axis of the illuminating light from the illuminating unit.

7. The capsule endoscope according to claim 1, wherein the illuminating light guide window is made of one of resin and glass.

8. The capsule endoscope according to claim 1, wherein a central portion of the front cover other than the illuminating light guide window is convex.

9. The capsule endoscope according to claim 1, wherein at least two illuminating units are arranged around the image capturing unit.

10. The capsule endoscope according to claim 1, further comprising a communication unit that transmits captured image information from the image capturing unit to outside.

11. A capsule endoscope system comprising:
the capsule endoscope according to claim 10;
a package that covers the capsule endoscope before the capsule endoscope is used;
a receiving unit that receives the information from the capsule endoscope; and
an information processing unit that processes the information received from the receiving unit.
